(19) Europäisches Patentamt
European Patent Office
Office européen des brevets

(11) Numéro de publication: **0 003 090**
**B1**

(12) # FASCICULE DE BREVET EUROPEEN

(45) Date de publication du fascicule du brevet: **20.01.82**

(21) Numéro de dépôt: **78400267.7**

(22) Date de dépôt: **28.12.78**

(51) Int. Cl.[3]: **C 07 J 1/00**, C 07 J 17/00, C 07 J 33/00, C 07 J 43/00, A 61 K 31/565, A 61 K 31/58 //C07J71/00

(54) **Nouveaux dérivés stéroides 17 alpha-aryle ou 17 alpha-hétéroaryle, leur procédé de préparation et leur application comme médicaments et les compositions pharmaceutiques les renfermant.**

(30) Priorité: **11.01.78 FR 7800658**

(43) Date de publication de la demande: **25.07.79 Bulletin 79/15**

(45) Mention de la délivrance du brevet: **20.01.82 Bulletin 82/3**

(84) Etats contractants désignés: **BE DE GB IT NL**

(56) Documents cités:
**DE - B - 1 046 040**
**FR - A - 1 360 436**

**TETRAHEDRON, vol. 21(1965), pages 1197—1202.**

(73) Titulaire: **ROUSSEL-UCLAF**
**102, route de Noisy Boîte postale no.9**
**F-93230 Romainville (FR)**

(72) Inventeur: **Pierdet, André**
**10, rue Charles Baudelaire**
**F-93130 Noisy le Sec (FR)**
Inventeur: **Torelli, Vesperto**
**5, rue de la Convention**
**F-94700 Maisons Alfort (FR)**
Inventeur: **Deraedt, Roger**
**23, allée Jean-Baptiste Clément**
**F-93320 Pavillons sous Bois (FR)**

(74) Mandataire: **Petranker, Léon et al,**
**boîte postale no 9 102, route de Noisy**
**F-93230 Romainville (FR)**

**EP 0 003 090 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés stéroides 17 alpha-aryle ou 17 alpha-hétéroaryle, leur procédé de préparation et leur application comme médicaments et les compositions pharmaceutiques les renfermant

La présente invention concerne de nouveaux dérivés stéroïdes 17 $\alpha$-aryle ou 17 $\alpha$-hétéroaryle, leur procédé de préparation, leur application comme médicaments et les compositions pharmaceutiques les renfermant.

L'invention a pour objet les composés de formule I:

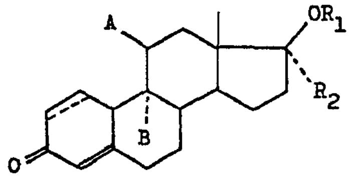

dans laquelle $R_1$ représente un atome d'hydrogène ou un radical acyle renfermant de 1 à 18 atomes de carbone, $R_2$ représente un radical aryle ou aralcoyle renfermant de 6 à 12 atomes de carbone éventuellement substitué, ou un radical hétéroaryle éventuellement substitué, choisi dans le groupe constitué par les radicaux furyle, thiényle, pyridyle et pyrimidyle, les traits pointillés dans le cycle A représentent une double liaison éventuelle en 1 (2) et, ou bien A représente un groupement-oxo et B représente un atome d'hydrogène, ou bien A représente un groupement-hydroxyle en position $\beta$ et B représente un atome d'halogène ou un atome d'hydrogène ou bien A et B forment ensemble une double liaison 9 (11).

Lorsque $R_1$ représente un radical acyle, il s'agit de préférence du reste d'un acide aliphatique ou cycloaliphatique saturé ou insaturé et notamment du reste d'un acide alcanoïque tel que l'acide acétique, propionique, butyrique ou isobutyrique ou undécylique, du reste d'un acide hydroxy-alconoïque tel que par exemple l'acide hydroxyacétique, du reste d'un acide cycloalcoylcarboxylique ou (cycloalcoyle) alcanoïque tel que l'acide cyclopropyl, cyclopentyl ou cyclohexylcarboxylique, cyclopentyle ou cyclohexyle acétique ou propionique, du reste d'un acide benzoïque ou d'un acide phényl alcanoïque tel que l'acide phénylacétique ou phényl propionique, du reste d'un aminoacide tel que l'acide diéthylamino acétique ou aspartique ou du reste de l'acide formique.

Lorsque $R_2$ représente un radical aryle ou aralcoyle, il s'agit de préférence du radical phényle ou benzyle, éventuellement substitué en ortho, méta ou para par un ou plusieurs radicaux hydroxy, par un ou plusieurs radicaux alcoyles renfermant de 1 à 4 atomes de carbone, par un ou plusieurs radicaux alcoxy renfermant de 1 à 4 atomes de carbone comme un radical méthoxy, par un ou plusieurs atomes d'halogène comme un atome de chlore ou de fluor, par un radical trifluorométhyle ou par une combinaison de ces divers substitutants.

Lorsque $R_2$ représente un radical hétéroaryle substitué, il s'agiot de préférence d'un radical hétéroaryle substitué par un ou plusieurs groupements hydroxy, par un ou plusieurs radicaux alcoyles renfermant de 1 à 4 atomes de carbone, par un ou plusieurs radicaux alcoxy renfermant de 1 à 4 atomes de carbone comme un radical méthoxy, par un ou plusieurs atomes d'halogène comme un atome de chlore ou de fluor, par un radical trifluorométhyle ou par une combinaison de ces divers substituants.

Lorsque B représente atome d'halogène, il s'agit de préférence d'un atome de fluor ou de chlore.

Parmi les composés de l'invention, ou peut citer les composés de formule I pour lesquels $R_2$ représente un radical aryle et notamment le radical phényle ou un radical aralcoyle et notamment le radical benzyle, éventuellement substitués par un groupement hydroxyle ou un groupement $CF_3$ et ceux pour lesquels $R_1$ représente un atome d'hydrogène. On peut citer également les composés de formule I pour lesquels A représente un groupement oxo et B un atome d'hydrogène, ceux pour lesquels A représente un groupement OH et B un atome d'hydrogène, ceux pour lesquels A représente un groupement OH et B un atome d'halogène comme par exemple un atome de fluor et ceux pour lesquels A et B forment ensemble une double liaison éthylénique en 9 (11).

On peut citer encore les composés de formule I pour lesquels le cycle A ne porte pas d'insaturation éthylénique en 1 (2) et ceux pour lesquels le cycle A porte une insaturation en 1 (2).

L'invention comprend plus spécifiquement les composés dont la préparation est donnée plus loin dans la partie expérimentale.

On peut citer parmi ceux-ci, la 11$\beta$, 17$\beta$-dihydroxy 17$\alpha$-o-hydroxyphényl androsta 1,4-diène-3-one.

L'invention a également pour objet un procédé de préparation des composés de formule I, caractérisé en ce que l'on soumet un composé de formule

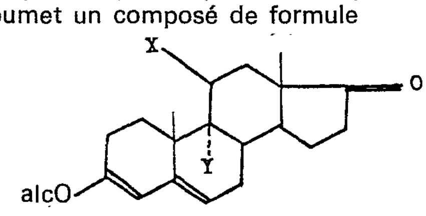

II

dans laquelle alc représente un radical alcoyle renfermant de 1 à 6 atomes de carbone, et, ou bien X représente un groupement oxo et Y représente un atome d'hydrogène, ou bien X et Y forment ensemble une double liaison éthylénique en 9 (11), à l'action d'un composé de formule $R_2$ Li, dans laquelle $R_2$ conserve la même signification que précédemment, puis à celle d'un agent d'hydrolyse acide, pour obtenir le composé de formule I A

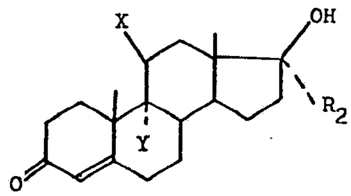

I A

— que l'on soumet si désiré, dans le cas où X représente un groupement oxo et Y représente un atome d'hydrogène, à l'action d'un orthoformiate d'alcoyle en milieu acide, puis à l'action d'un agent de réduction et enfin à celle d'un agent d'hydrolyse acide, pour obtenir un composé de formule

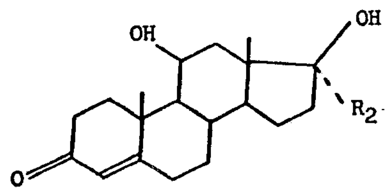

I B

— ou que l'on soumet, dans le cas où X et Y forment ensemble une double liaison éthylénique en 9 (11), à l'action du N-bromosuccinimide en présence d'acide perchlorique, pour obtenir un composé de formule

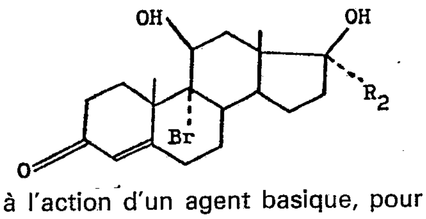

I C

que l'on soumet, si désiré, à l'action d'un agent basique, pour obtenir un composé de formule

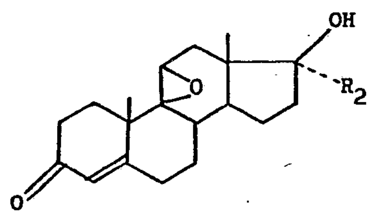

III

que l'on soumet à l'action d'un hydracide de formule H Hal dans laquelle Hal représente un atome de chlore ou de fluor, pour obtenir un composé de formule:

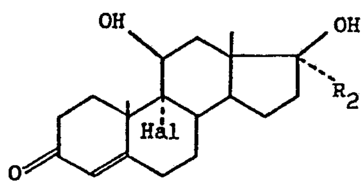

I D

puis, si désiré, soumet chacun des composés obtenus IA, IB, IC ou ID à l'action d'un agent de deshydrogénation, pour obtenir les composés Δ 1,4 correspondants, et soumet enfin, si désiré, chacun des composés obtenus, répondant à la formule I dans laquelle $R_1$ représente un atome d'hydrogène, à l'action d'un agent d'estérification sélectif, pour obtenir les composés correspondants répondant à la formule I dans laquelle $R_1$ représente un radical. acyle tel que défini. Dans un mode de réalisation préférée du procédé de l'invention, la réaction entre le composé de formule II et le composé de formule $R_2$ Liest réalisée au sein de l'éther éthylique ou du tétrahydrofurane ou au sein d'un mélange d'éther éthylique et de tétrahydrofurane et l'on utilise comme agent d'hydrolyse acide, l'acide chlorhydrique, sulfurique, acétique, citrique ou para toluène sulfonique, l'hydrolyse pouvant être effectuée au sein d'un ou plusieurs solvants tel qu'un alcool comme le méthanol, l'éthanol ou l'isopropanol, une cétone comme l'acétone. On utilise comme orthoformiate d'alcoyle, l'orthoformiate de méthyle ou d'éthyle, comme agent de réduction un borohydrure alcalin comme le borohydrure de sodium ou de potassium ou

bien l'hydrure mixtede lithium et d'aluminium et comme agent basique susceptible de transformer le composé de formule IC en composé de formule III, la potasse ou la soude ou un alcoolate alcalin comme le méthylate ou l'éthylate de sodium. Pour transformer les composés des formules IA, IB, IC ou ID en composés correspondants portant une double liaison en 1 (2) on utilise la voie biochimique, notamment au moyen de la bactérie arthrobacter simplex, ou encore des dérivés de la p-benzoquinone comme la 2,3-dichloro 5,6- dicyanobenzoquinone.

Comme agent d'estérification, un utilise de préférence un anhydride ou un chlorure d'acide et l'on opère en présence d'un catalyseur acide.

Les produits de formule II sont en général connus, ils peuvent être préparés, par exemple, selon le procédé indiqué dans le brevet américain 3 055 917.

Les 3-alcoxy androsta 3,5,9 (11)-triène 17ones et notamment le 3-éthoxy androsta 3,5,9 (11)-triène 17-one sont des produits nouveaux. Ils peuvent être préparés par action d'un orthoformiate d'alcoyle sur l'androsta 4,9 (11)-diène 3,17-dione.

L'invention a également pour objet une variante du procédé défini ci-dessus, caractérisé en ce que le composé de formule III est obtenu en soumettant un composé de formule

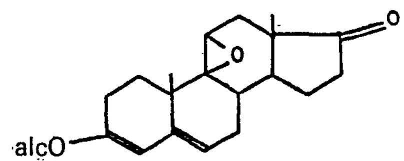

IV

dans laquelle alc représente un radical alcoyle renfermant de 1 à 6 atomes de carbone, à l'action d'un composé de formule $R_2$ Li dans laquelle $R_2$ conserve la même signification que précédemment, puis à celle d'un agent d'hydrolyse acide, pour obtenir le composé de formule III désiré.

La réaction du composé de formule IV avec le compsé $R_2$ Li est réalisée de préférence dans les conditions décrites ci-dessus pour le composé de formule II.

Les composés de formule IV et notamment le 3-éthoxy 9β, 11β époxy androsta 3,5-diène 17-one sont des produits nouveaux. Ils peuvent être préparés par action d'un orthoformiate d'alcoyle sur le 9β, 11β-époxy androst-4-ène 3,17-dione (produit connu et décrit notamment dans le brevet des Etats-Unis d'Amérique 3 072 643).

Les composés de formule I présentent d'intéressantes propriétés pharmacologiques et notamment une très bonne activité anti-inflammatoire.

L'invention a donc également pour objet l'application, à titre de médicaments, des composés de formule I, et notamment de la 11β, 17β-dihydroxy 17α-o-hydroxyphényl androsta 1,4-diène 3-one.

Ces composés peuvent être utilisés en thérapeutique notamment dans le traitement des affections rhumatismales, des arthroses, lombalgies, sciatiques, névralgies, myalgies ou des douleurs dentaires.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés, simplesou dragéifiés, les gélules, les granulés, les suppositoires ou les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants et les conservateurs.

La posologie varie notamment en fonction de la voie d'administration, de l'affection traitée et du sujet en cause.

Par exemple, chez l'adulte, elle peut varier entre 5 mg et 100 mg de principe actif par jour, par voie buccale pour le composé de l'exemple 3.

Il va être donné maintenant, à titre non limitatif des exemples de mise en oeuvre de l'invention.

Préparation 1

*3-éthoxy androsta 3,5,9 (11)-triène 17-one.*

Dans 74 cm3 d'éthanol, on introduit 22,08 g d'androsta 4,9 (11)-diène 3,17-dione, puis 60 cm3 d'orthoformiate d'éthyle, chauffe le mélange à 75°C.

Après dissolution, on ajoute 28,6 cm3 d'une solution éthanolique contenant 18,4 mg d'acide paratoluène sulfonique, agite 3 minutes puis ajoute 3,7 cm3 de triéthylamine. On refroidit, dilue avec de l'eau et extrait avec du chlorure de méthylène. On sèche la phase organique sur sulfate de sodium,

concentre à sec et purifie l'extrait sec, par chromatographie sur silice en éluant avec le mélange cyclohexane — acétate d'éthyle (9—1) — triéthylamine: 1%.

On isole 18,8 g de produit pur cristallisé. PF: 131°C.

*Spectre I.R.* (chloroforme) >C = O 1736 $cm^{-1}$

*Spectre U.V.* (Ethanol) Max: 241 nm $\varepsilon = 16200$

Préparation 2

*3-éthoxy 9β, 11β-époxy androsta 3,5-diène 17-one*

Dans 66 cm3 d'éthanol, on introduit 20 g de 9β, 11β-époxy androsta 4-ène 3,17-dione puis 54 cm3 d'orthoformiate d'éthyle, chauffe la mélange à 72°, 74°C. Après dissolution, on ajoute en 3 fois à 5 minutes d'intervalle 60 cm3 d'une solution éthanolique d'acide paratoluène sulfonique à 0,8% en maintenant la température à 72°C—74°C. On refroidit le mélange, introduit 2 cm3 de triéthylamine, verse dans l'eau, extrait au chlorure de méthylène, lave à l'eau saturée de chlorure de sodium, sèche la phase organique sur sulfate de sodium et concentre à sec sous pression réduite à 40°C. L'extrait sec est purifié par chromatographie sur silice en éluant successivement avec les mélanges

Essence B (Eb 60—80°C) benzène (8—2) à 1% de triéthylamine

Essence B benzène (6—4) à 1% de triéthylamine

Essence B benzène (2—8) à 1% de triéthylamine

On isole 15,5 g de produit pur.

*Spectre I.R.* (chloroforme)

>C = O 1740 $cm^{-1}$

Δ 3,5 + 3 éther 1652 $cm^{-2}$ et 1628 $cm^{-1}$

Exemple 1

*17β-hydroxy 17α-o-hydroxyphenyl androst 4-ène 3,11-dione*

Dans 360 cm3 d'éther éthylique anhydre, on introduit, sous atmosphère inerte sèche, 26,8 g d'orthobromophénol, agite la solution à —5°C et ajoute goutte à goutte 180 cm3 d'une solution 2M de butyllithium dans l'hexane. On porte ensuite au reflux pendant 1 heure.

On refroidit le mélange réactionnel à —20°C, ajoute en 20 minutes 30,8 g de 3-éthoxy androsta 3,5-diène 11,17-dione, laisse réagir 1 heure à la température ambiante, puis verse le mélange dans de l'eau glacée, ajoute de l'acide chlorhydrique et extrait avec de l'acétate d'éthyle. On lave, sèche, évapore le solvant. Le résidue huileux dissous dans 200 cm3 d'éthanol absolu est chauffé à 60°C pendant 20 minutes avec 40 cm3 d'acide sulfurique N. On refroidit, dilue à l'eau et extrait avec de l'acétate d'éthyle. L'extrait sec est purifié chromatographie sur silice en éluant avec le mélange benzène-acétate d'éthyle (8—2). Après séparation, on recueille 14,2 g de produit que l'on cristallise dans l'éthanol aqueux. On glace, essore, lave avec de l'éthanol à 50% d'eau, sèche et obtient 10,78 g de produit pur. PF = 295°C.

*Analyse:* $C_{25}H_{30}O_4 = 394,51$ calculé C% 76,11 H% 7,66
trouvé 76,1 7,7

| | | |
|---|---|---|
| *Spectre I.R.* (chloroforme) | C = O | 1709 $cm^{-1}$ |
| | C =O conjugué | 1670 $cm^{-1}$ |
| | C = C | 1619 $cm^{-1}$ |
| | aromatique | 1584 $cm^{-1}$ 1491 $cm^{-1}$ |
| | OH | 3564 $cm^{-1}$ |

*Spectre UV* (Ethanol)

Max 228 nm $\varepsilon = 15900$

Max 238 nm $\varepsilon = 15800$

Max 274 nm $\varepsilon = 2760$

Infl 280 nm

Infl 310 nm

Exemple 2

*11β, 17β-dihydroxy 17α-o-hydroxy phényl androst 4-ène 3-one*

Dans 25 cm3 d'éthanol, on introduit 2,53 g de 17β-hydroxy 17α-ortho hydroxyphényl androst 4-ène 3,11-dione préparé à l'exemple 1 et 2,5 cm3 d'orthoformiate d'éthyle.

On porte au reflux et ajoute 0,5 cm3 d'une solution d'acide sulfurique à 0,2 ml dans 100 ml d'éthanol, agite 3 minutes puis ajoute 1 cm3 de triéthylamine, refroidit, dilue à l'eau, extrait avec du chloroforme, lave, sèche et évapore le solvant sous pression réduite. On obtient le 3 éthoxy, 17β-hydroxy, 17α-o-hydroxyphényl androst 3,5-diène 11-one sous formé de résine. Le produit brut est dissout dans 10 cm3 d'éthanol et 4 cm3 de soude 2N. On ajoute 5 g d'hydroborure de sodum, et chauffe le mélange au reflux pendant 4 heures. On refroidit, verse dans de l'eau glacée additionnée d'acide chlorhydrique, extrait avec du chloroforme, lave à l'eau, sèche et évapore le solvant sous pression réduite. Le résidu est d'abord chauffé à 45°C pendant 10 minutes avec 20 cm3 d'éthanol et 5 cm3 d'acide chlorhydrique 2 N. On ajoute ensuite 1g d'acétate de sodium 2 cm3 d'acide acétique, maintient 15 minutes à 40°C puis dilue à l'eau et extrait avec du chloroforme, lave et évapore le solvant. L'extrait sec est purifié par chromatographie sur silice (benzène-acétate d'éthyle 7—3). On obtient 2,1 g du produit attendu, fondant à 276°C après recristallisation dans l'isopropanol.

*Analyse* $C_{25}H_{32}O_4$ = 396,53 calculé C% = 75,72 H% = 8,13
trouvé 75,8 8,2

*Spectre IR* (chloroforme) OH: 3608 $cm^{-1}$ 3580 $cm^{-1}$ 3300 $cm^{-1}$

*Spectre UV* (ethanol) infl 228 nm ε = 13600

max 241—242 nm ε = 15300

infl 272 nm

infl 280 nm

infl 305 nm

Exemple 3

*11β, 17β-dihydroxy 17α-o-hydroxyphenyl androsta 1,4-diène 3 one.*

Dans 750 ml d'une solution tampon pH = 7 composée de 9,6 g de phosphate monopotassique, 32 cm3 de soude N et 1400 cm3 d'eau, on introduit 15 g d'arthrobacter simplex (poudre ATCC 6946) 1,5 g de silice diatomée. On ajoute une solution de 780 mg de 11β, 17β-dihydroxy 17α-o-hydroxyphenyl androst 4-ène 3 one préparée à l'exemple 2 dans 15 cm3 de méthanol et maintient 40 heures à 35°C ± 1°C sous barbotage d'air. On filtre, extrait avec du chloroforme lave à l'eau, sèche, concentre à sec et obtient 1g de résidu sec cristallisé que l'on chromatographie sur silice (éluant chloroforme à 2% de méthanol). On isole 480 mg de produit attendu par recristallisation dans un mélange de chlorure de méthylène et d'éther isopropylique PF = 264°C.

*Analyse* $C_{25}H_{30}O_4$ = 394,51 calculé C% = 76,11 H% = 7,66
trouvé 76,1 7,8

*Spectre IR* (chloroforme) C = O 1663 $cm^{-1}$

système conjugué C = C 1623 $cm^{-1}$ 1606 $cm^{-1}$ 1583 $cm^{-1}$

OH 3606 $cm^{-1}$ 3587 $cm^{-1}$

*Spectre UV* (éthanol)

Max à 225 nm ε 13400

Max à 243 ε 14250

Infl à 308 nm

Exemple 4

*17α-phényl, 17β-hydroxy androsta 4,9 (11)-diène 3-one*

Stade A: 3 éthoxy 17α-phényl 17β-hydroxy androsta 3,5,9 (11)-triène

Dans 25 cm3 d'ether éthylique, on introduit 4,7 cm3 de phényllithium (titre 1,37 mol/l), chauffe au reflux et ajoute 500 mg de 3-éthoxy androsta 3,5,9 (11)-trien 17-one obtenu à la préparation 1 agite 1 heure 30 sous atmosphère inerte, en maintenant la température de reflux, ajoute 13,6 cm3 d'éther éthylique saturé d'eau, agite 25 minutes, ajoute 4,7 $cm^3$ de phényllithium, agite 1 heure, ajoute de nouveau 10 cm3 d'éther éthylique saturé d'eau, agite 15 Minutes, ajoute 14 cm3 de phényllithium et agite encore 1 heure, refroidit, ajoute une solution aqueuse de chlorure d'ammonium, lave à l'eau la phase organique, sèche, concentre à sec sous pression réduite, obtient 1,9 g de produit attendu.

Stade B: 17α-phényl, 17β-hydroxy androsta, 4,9 (11)-diene 3-one

Au produit obtenu au stade précédent, on ajoute 19 cm3 d'éthanol, 1,9 cm3 d'acide sulfurique N, chauffe à 60°C en agitant sous atmosphère inerte, pendant 30 minutes, refroidit, neutralise à l'aide d'une solution aqueuse de bicarbonate de sodium. On dilue au chlorure de méthylène, lave à l'eau saturée de chlorure de sodium, sèche et évapore à sec le solvant, ajoute 5 cm3 d'éther isopropylique, glace encore, lave à l'éther isopropylique, sèche et obtient 360 mg de produit PF = 244°C.

Par chromatographie sur silice des liqueurs mères (éluant benzène acétate d'éthyle 8—2) suivie d'une cristallisation dans l'éther isopropylique, on obtient encore 93 mg de produit PF = 244°C.

*Spectre I.R.* (chloroforme)

| | | | |
|---|---|---|---|
| Système conjugué + aromatique | C = O | 1663 $cm^{-1}$ | |
| | C = C | 1613 $cm^{-1}$ | et 1593 $cm^{-1}$ |
| | OH | 3593 $cm^{-1}$ | |

Exemple 5

*17β-hydroxy 17α o-hydroxy phényl androsta 4,9 (11)-dien 3-one*

Stade A: 3-éthoxy 17α o-hydroxyphényl 17β-hydroxy androsta 3,5,9 (11)-triène.

Dans 480 cm3 d'éther éthylique, on introduit 24 g d'orthobromophénol, refroidit à une température comprise entre 0°C et +5°C en agitant sous atmosphère inerte, introduit en 10 minutes 171 cm3 d'une solution de butyllithium dans l'hexane (1,55 mole/litre), chauffe au reflux 1 heure, refroidit et obtient le lithien cherché à 0,20 mole/litre.

On chauffe au reflux 90 cm3 de réactif préparé ci-dessus et introduit une solution de 1,4 g de 3-éthoxy androsta 3,5,9 (11)-triène 17-one obtenu à la préparation 1 dans 18 cm3 d'éther éthylique, on agite au reflux 1 heure 30 sous atmosphère inerte puis en 2 fois consécutives, adjoute 56 cm3 d'éther éthylique saturé d'eau, agite 10 minutes, ajoute 90 cm3 de lithien, agite 45 minutes. On termine en ajoutant 28 cm3 d'éther éthylique saturé d'eau, agite 10 minutes et en ajoutant 180 cm3 de lithien, agite encore 15 heures au reflux. On refroidit le mélange à une température comprise entre 0°C et 5°C, ajoute 100 cm3 d'une solution aqueuse saturée de chlorure d'ammonium, sépare la phase organique, lave à l'eau, sèche sur sulfate de sodium, concentre à sec sous pression réduite à 40°C et obtient 14 g de produit attendu sous forme d'huile.

Stade B: 17β-hydroxy 17α-o-hydroxyphényl androsta 4,9 (11)-dien 3-one

Au produit obtenu au stade précédent, on ajoute 70 cm3 d'éthanol, 14cm3 d'acide sulfurique N, chauffe à 60°C et agite pendant 30 minutes sous atmosphère inerte, refroidit, ajoute 20 cm3 de solution aqueuse saturée de bicarbonate de soude, ajoute 50 cm3 d'acétate d'éthyle, sépare par décantation, lave à l'eau saturée de chlorure de sodium, sèche, concentre à sec et obtient 10,7 g de produit brut. On purifie par chromatographie, sur silice en éluant avec du benzène, puis avec un mélange benzène-acétate d'éthyle 8—2. On isole le produit, ajoute 8 cm3 de benzène, essore, lave au benzène, sèche et obtient 1,03 g de produit pur. PF ≃ 260°C. Par chromatographie sur silice des liqueurs mères (éluant benzène acétate d'éthyle 9—1) on obtient encore 120 mg de produit pur. PF = 260°C.

*Analyse:* $C_{25}H_{30}O_3$ = 378,49

| | | C% | H% |
|---|---|---|---|
| Calculé | | 79,32 | 7,98 |
| Trouvé | | 79,0 | 8,00 |

*Spectre I.R.* (chloroforme)

| | | | | |
|---|---|---|---|---|
| | OH = | 3610 $cm^{-1}$ | 3580 $cm^{-1}$ | |
| Système conjugué + aromatique | C = O | 1665 $cm^{-1}$ | | |
| | C = C | 1618 $cm^{-1}$ | 1582 $cm^{-1}$ | 1490 $cm^{-1}$ |

*Spectre UV* (éthanol)

infl 230 nm $E_1^1 = 379$

max 239 nm $E_1^1 = 405$ $\varepsilon = 15300$

$[\alpha]_D = \pm 148° \pm 3$ (0,5% chloroforme)

Exemple 6

*9α-fluoro 11β, 17β-dihydroxy 17α-o-hydroxyphényl androst 4-en 3-one.*

Stade A: 9β, 11β-époxy 17β-hydroxy 17α-o-hydroxyphényl androst 4-en 3-one.

On chauffe au reflux 234 cm3 de lithien préparé comme décrit à l'exemple 5 et sous atmosphère inerte, ajoute une solution de 4,5 g de 3-éthoxy 9β 11β-époxy androsta 3,5-diène 17-one obtenu à la préparation 2 dans 45 cm3 d'éther éthylique anhydre agite 1 heure au reflux. Le milieu est ensuite soumis à des protonations successives alternant avec des introductions nouvelles de réactif. On procède de la façon suivante:

on ajoute 171 cm3 d'éther éthylique saturé d'eau et porte au reflux 10 minutes,
on ajoute 351 cm3 de lithien et porte au reflux 45 minutes,
on ajoute 86 cm3 d'éther éthylique saturé d'eau et porte au reflux 10 minutes,
on ajoute 334 cm3 de lithien et porte au reflux 15 heures.

On glace, ajoute 300 cm3 d'une solution aqueuse saturée de chlorure d'ammonium, sépare par décantation la phase organique, lave à l'eau et à l'eau saturée de chlorure de sodium, sèche, concentre à sec et obtient 15 g de 3-éthoxy, 9β, 11β-époxy 17α-o-hydroxyphényl 17β-hydroxy androsta 3,5-diène. On ajoute 75 cm3 d'éthanol, 15 cm3 d'acide sulfurique N, chauffe 30 minutes à 60°C sous agitation et atmosphère inerte. On refroidit, verse dans 100 cm3 d'une solution aqueuse saturée de bicarbonate de sodium, ajoute 100 cm3 d'acétate d'éthyle, sépare par décantation, lave à l'eau saturée de chlorure de sodium, sèche, concentre à sec sous pression réduite et obtient 14,3 g de produit brut. On purifie par chromatographie sur silica en éluant avec du benzène puis avec un mélange benzène acétate d'éthyle 8—2. On isole le produit cristallisé par addition de benzène et obtient 2,93 g du produit attendu. Par chromatographie sur silice des liqueurs mères, en éluant avec un mélange d'éthyle 7—3 et cristallisation dans le benzène, on obtient un deuxième jet de 0,32 g.
P.F. = 170°C.

| | | | | |
|---|---|---|---|---|
| *Spectre I.R.* | (chloroforme) | OH 3608 $cm^{-1}$ | 3576 $cm^{-1}$ | |
| | aromatique | 1584 $cm^{-1}$ | 1490 $cm^{-1}$ | |
| | C=C | 1622 $cm^{-1}$ | | |
| | Δ 4,3-one | 1669 $cm^{-1}$ | 1662 $cm^{-1}$ | 1651 $cm^{-1}$ |

| | | | | |
|---|---|---|---|---|
| *Spectre U.V.* | (éthanol) | max. 225 nm | $E_1^1 = 270$ | |
| | | max. 244—245 nm | $E_1^1 = 352$ | ($\varepsilon$ 13900) |
| | | infl. 279 nm | $E_1^1 = 60$ | |

Stade B: 9α-fluoro 11β, 17β-dihydroxy 17α-o-hydroxyphényl androst 4-en 3-one.

On refroidit à −35°C 11,5 cm3 de complexe HF-diméthylformamide et introduit en 10 minutes, sous agitation, 5,77 g de 9β 11β-époxy 17β-hydroxy 17α-o-hydroxyphényl androst 4-ène 3-one obtenu au stade précédent, agite 25 minutes, verse le mélange réactionnel dans 200 cm3 d'un mélange d'eau, de glace et de 35 cm3 d'ammoniaque, agite 30 minutes, essore, lave à l'eau jusqu'à neutralité, sèche, obtient 5,83 g de produit brut, ajoute 20 cm3 de chlorure de méthylène, chauffe 15 minutes au reflux, refroidit, essore, lave au chlorure de méthylène, sèche et obtient 4,05 g de produit pur. P.F. = 260°C. après recristallisation sur bloc Maquenne à 195°C.

| | | | | | |
|---|---|---|---|---|---|
| Analyse: $C_{25}H_{31}O_4F$ 414,50 | Calculé: | C% 72,43 | H% 7,47 | F% 4,58 |
| | Trouvé: | 72,5 | 7,6 | 4,5 |

$[\alpha]_D = +125° \pm 3°5$ (0,6% EtOH)

*Spectre U.V.* (éthanol) infl. 230 nm $E_1^1 = 342$

infl. 240 nm $E_1^1 = 368$ $\varepsilon = 15250$

max. 273 nm $E_1^1 = 56$ $\varepsilon = 2300$

infl. 278 nm $E_1^1 = 50$

Spectre U.V. (éthanol/Na OH N/10)

maxi 241 $\varepsilon$ 22300

293 $\varepsilon$ 3800

*Spectre IR* (nujol)

système conjugué + aromatiques { C = O 1657 $cm^{-1}$ ; C = C 1619 $cm^{-1}$ 1580 $cm^{-1}$ 1495 $cm^{-1}$

OH 3531 $cm^{-1}$

Exemple 7

*9α-fluoro 11β, 17β-dihydroxy 17α-o-hydroxyphényl androsta 1,4-dien 3-one.*

On dissout 27,6 g de phosphate de potassium monobasique dans 3,95 litres d'eau distillée et ajuste à pH = 7 à laide de 11,2 cm3 de lessive de soude 10 N. Dans 3,1 litres de cette solution chauffée à 42°C, on introduit en 3 minutes 3,4 g de 9α fluoro, 11β, 17β-dihydroxy, 17α o-hydroxyphényl androst 4-ène 3-one préparé à l'exemple 6, dissous dans 184 cm3 de méthanol. On maintient la température et un léger barbotage d'air, introduit à 3 reprises 24 g de silice diatomée et 24 g d'arthrobacter simplex (poudre ATCC 6 946) en agitant 30 heures après la lère introduction, 100 heures après la 2ème, 18 heures après la 3ème. On essore sur silice diatomée, extrait le filtrat à l'acétate d'éthyle puis au chlorure de méthylène; on isole les phases organiques, sèche sur sulfate de sodium et concentre à sec sous pression réduite; on ajoute au résidu 15 cm3 de chlorure de méthylène, essore, lave au chlorure de méthylène, sèche et obtient 2,22 g de produit qui est purifié par chromatographie sur silice (éluant chloroforme dioxanne méthanol 9—10—1) et cristallisation dans le chlorure de méthylène, on obtient 1,61 g de produit pur. P.F. = 208°C.

*Analyse*

| | | | | |
|---|---|---|---|---|
| $C_{25}H_{29}O_4F$ PM = 412,48 | calculé % = | 72,79 | H% = 7,08 | F% = 4,60 |
| | trouvé | 72,9 | 7,1 | 4,8 |

$[\alpha]_D = +134° \pm 3$ (éthanol 0,5%)

*Spectre UV* (éthanol) max = 226 nm $E_1^1 = 350$ $\varepsilon = 14400$

max = 238 nm $E_1^1 = 354$ $\varepsilon = 14600$

infl = 270 nm $E_1^1 = 153$ $\varepsilon = 6300$

infl = 280 nm $E_1^1 = 84$ $\varepsilon = 3500$

*Spectre I.R.* (nujol) Système conjugué { C = O 1658 $cm^{-1}$ ; C = C 1613 $cm^{-1}$ 1600 $cm^{-1}$ 1580 $cm^{-1}$

Δ1,4,3one 886 $cm^{-1}$

Exemple 8

*9α-bromo 11β, 17β-dihydroxy 17α phényl androst-4-en 3-one.*

Dans 103 cm3 d'acétone anhydre refroidi à une température comprise entre 0°C et +5°C, on introduit sous atmosphère inerte 4,36 g de N bromosuccinimide et 6,83 g de 17α-phényl 17β-hydroxy androsta — 4,9 (11)-diène 3-one obtenu à l'exemple 4 B. On ramène la température entre +5°C et 10°C, ajoute en 25 minutes une solution de 21 cm3 d'eau et 4,36 cm3 d'acide perchlorique à 55° Bé, agite 10 minutes, dilue avec 200 cm3 d'eau, essore, lave à l'eau jusqu'à neutralité et obtient le produit attendu.

Exemple 9

*9α-fluoro 11β, 17β-dihydroxy 17α-phényl androst 4-en 3-one.*

Stade A: 9β, 11β-époxy 17β-hydroxy 17α phényl androst-4-ène 3-one.

Au 9 α-bromo 11β, 17β-dihydroxy 17α-phényl androst-4-en 3-one préparé selon l'exemple 8, on ajoute 27,5 cm3 de potasse méthanolique N, 41 cm3 de méthanol, agite 1 heure 30 à 20°C—25°C sous atmosphère inerte, refroidit, dilue le mélange réactionnel avec 150 cm3 d'eau et de glace, essore, lave à l'eau jusqu'à neutralité, sèche et obtient 6,14 g de produit brut. On ajoute 150 cm3 d'isopropanol, chauffe au reflux jusqu'à dissolution, filtre à chaud, concentre à 100 cm3, glace, essore, lave à l'isopropanol glacé, sèche obtient 4,277 g de produit pur d'un premier jet puis 0,793 g dans un deuxième jet. PF = 216°C.

$[\alpha]_D = +3° \pm 2°$ (chloroforme 0,5%)

| | | | | |
|---|---|---|---|---|
| *spectre UV* | (éthanol) | infl 215 nm | $E_1^1 = 268$ | |
| | | infl 220 nm | $E_1^1 = 245$ | |
| | | max 244 nm—254 nm | $E_1^1 = 367$ | $\varepsilon = 13900$ |

*spectre IR* (chloroforme)

| | |
|---|---|
| OH 3595 $cm^{-1}$ | =C−H de $\Delta_4$+ époxy 865 $cm^{-1}$ |
| C=O 1660 $cm^{-1}$ | aromatique 1600—1492 $cm^{-1}$ |
| C=C 1619 $cm^{-1}$ | |

Stade B: 9α-fluoro 11β, 17β-dihydroxy 17α phényl androst 4-en 3-one

Dans 18,8 cm3 de complexe acide fluorhydrique-diméthylformamide refroidi à une température comprise entre −30°C et −35°C, on introduit sous atmosphère inerte 11,4 g de 9β 11β-époxy 17β-hydroxy 17α-phényl androst 4 ène 3 one préparé au stade précédent, agite pendant 5 heures à cette température, verse le mélange dans 80 cm3 d'ammoniaque concentrée additionnée de 160 g de glace, agite 15 minutes, essore, lave à l'eau jusqu'à neutralité, sèche et obtient 15,6 g de produit brut. On ajoute 25 volumes d'acétone à 20% d'eau chauffe au reflux 30 minutes sous atmosphère inerte en présence de 1,56 g de charbon actif. On filtre sur silice diatomée, concentre, glace, essore, lave 2 fois à l'aide de 15 cm3 d'acétone à 60% d'eau glacée, sèche et isole 4,37 g de produit que l'on met en solution dans 800 cm3 de chlorure de méthylène; on ajoute 0,5 partie de silicate de magnésium, agite pendant 1 heure à 20°C sous atmosphère inerte, filtre, lave 2 fois avec 10 cm3 de chlorure de méthylène, chasse le solvant, sèche et reprend le résidu dans 20 volumes de diméthoxypropane, chauffe au reflux; filtre à chaud, concentre, glace, essore, lave 2 fois à l'aide de 10 cm3 de diméthoxypropane glacé, sèche et obtient 3,19 g de produit attendu. Le traitement analogue des liqueurs mères permet d'obtenir encore 2,53 g de produit attendu. PF = 240°C.

*Analyse*

$C_{25}H_{31}O_3F = 398,5$

| | | | |
|---|---|---|---|
| calculé | C% = 75,35 | H% = 7,84 | F% = 4,76 |
| trouvé | 75,0 | 8,1 | 4,6 |

$[\alpha]_D^{20} = +93° \pm 3°$ (chloroforme 0,5%)

| | | | |
|---|---|---|---|
| *spectre I.R.* (chloroforme) | C=O | 1665 $cm^{-1}$ | |
| | C=C | 1625 $cm^{-1}$ | |
| | φ | 1493 $cm^{-1}$ | |
| | OH | 3610 $cm^{-1}$ | et 3580 $cm^{-1}$ |

| | | | |
|---|---|---|---|
| *spectre UV* (éthanol) | Max 216—217 nm | $E_1^1 = 310$ | |
| | max 238—239 nm | $E_1^1 = 408$ | $\varepsilon = 16300$ |
| | infl 258 nm | $E_1^1 = 5$ | |
| | infl 320 nm | $E_1^1 = 2$ | |

Exemple 10

*9α-fluoro 11β, 17β-dihydroxy 17α-phényl androsta 1,4-diène 3-one.*

On chauffe à 32°C 2050 cm3 d'une solution tampon pH = 7, préparée selon l'exemple 7, sous agitation et barbotage d'air comprimé, on introduit une solution de 2,25 de 11β, 17β dihydroxy 9α fluoro 17α phényl androst 4-ène 3-one, dissous dans 122 cm3 de méthanol; on ajoute 15 g de silice diatomée et 15 g d'arthrobacter simplex (poudre ATCC 6946), et renouvelle cette opération après 25 heures puis après 116 heures de réaction. Entre chaque addition, on ajuste le mélange à pH = 7 à l'aide de phosphate de potassium monobasique en solution saturée. Après 187 heurs de réaction, on décante, extrait à 2 reprises au chlorure de méthylène, essore sur silice diatomée, extrait la phase aqueuse à 2 reprises à l'acétate d'éthyle, réunit les phases organiques, sèche et concentre à sec sous pression réduite. On purifie par chromatographie sur silice en éluant avec le mélange chloroforme-méthanol (100—5), isole le produit attendu, qui est cristallisé dans le mélange chlorure de méthylène-éther isopropylique et obtient 1,22 g de produit pur dans un premier jet et 0,23 mg dans un deuxième jet. On obtient le produit pur non solvaté après recristallisation dans le mélange chlorure de méthylène-diméthoxy-propane. PF = 268°C.

$[\alpha]_D = +95°$ (0,5% éthanol)

*Analyse*

| | | | | |
|---|---|---|---|---|
| $C_{25}H_{29}O_3F = 396,48$ | Calculé: | C% = 75,72 | H% = 7,37 | F% = 4,79 |
| | Trouvé: | 75,9 | 7,5 | 4,7 |

*Spectre IR.*

| | | |
|---|---|---|
| OH | 3608 $cm^{-1}$ | 3590 $cm^{-1}$ |
| C = O | 1667 $cm^{-1}$ | |
| C = C | 1627 $cm^{-1}$ | 1609 $cm^{-1}$ |
| = C—H | 890 $cm^{-1}$ | |
| aromatique | 1502 $cm^{-1}$ | |

*Spectre UV*

| | | |
|---|---|---|
| Max 216 nm | $E_1^1 = 330$ | $\varepsilon = 13000$ |
| infl. 219 nm | $E_1^1 = 325$ | |
| max 237—238 nm | $E_1^1 = 375$ | $\varepsilon = 14900$ |

Exemple 11

*9α-fluoro 11β, 17β-dihydroxy 17α-[(3-trifluoro méthyl) phényl]androst 4-en 3-one*

Stade A: 9β, 11β époxy 17α[(3-trifluorométhyl) phényl]17β hydroxy androst 4-ène 3-one.

Dans 357 cm3 d'éther éthylique anhydre, on introduit en 10 minutes 357 cm3 d'une solution de butyllithium dans le cyclohexane (titre 2,1 mole/litre), refroidit à 5°C, introduit en 1 heure 50 minutes une solution de 165 g de trifluorométhylbromobenzène dans 330 cm3 d'éther éthylique, agite deux heures, puis laisse revenir à température ambiante en maintenant l'agitation pendant trois heures, obtient un réactif lithien dont le titre est 0,5 mole/litre. On chauffe au reflux 212 cm3 de lithien, ajoute une solution de 8,7 g de 3-éthoxy 9β 11β-époxy androsta 3,5-diène 17-one obtenu à la préparation 2 dans 103 cm3 d'éther éthylique anhydre, agite 1 heure au reflux. Le milieu est ensuite soumis à des protonations succesives alternant avec des introductions nouvelles de réactif. On procède de la façon suivante:

on ajoute 226 cm3 d'éther éthylique saturé d'eau et agite au reflux 5 minutes,
on ajoute 212 cm3 de lithien et agite au reflux 1 heure
on ajoute 226 cm3 d'éther éthylique saturé d'eau et agite au reflux 5 minutes,
on ajoute 424 cm3 de lithien et agite au reflux pendant 15 heures.

On refroidit à 10°C, ajoute 100 cm3 d'une solution aqueuse saturée de chlorure d'ammonium. On isole la phase organique par décantation, lave à l'eau et à l'eau saturée de chlorure de sodium, sèche, concentre à sec et obtient 48 g de 3-éthoxy 9β 11β époxy 17α[(3-trifluorométhyl) phényl] 17β hydroxy androsta 3,5 diène.

On ajoute 480 cm3 d'éthanol et 48 cm3 d'acide sulfurique N, chauffe 30 minutes à 60°C en agitant sous atmosphère inerte, refroidit à 10°C et neutralise à l'aide d'une solution aqueuse saturée de bicarbonate de soude. On décante, ajoute 500 cm3 de chlorure de méthylène, lave à l'eau saturée de chlorure de sodium, sèche et concentre à sec pour obtenir 39 g de produit brut que l'on purifie par

chromatographie sur silice en éluant avec le mélange benzène-acétate d'éthyle 7—3, ajoute 150 cm3 d'éther isopropylique, essore, lave avec ce même solvant, sèche et isole 9,1 g de produit attendu PF: 146°C.

*Spectre I.R.* (chloroforme) OH 3605 $cm^{-1}$ 3590 $cm^{-1}$

aromatique 1485 $cm^{-1}$

$CF_3$ 1328 $cm^{-1}$ 1167 $cm^{-1}$ 1128 $cm^{-1}$

*Spectre U.V.* (éthanol) infl 218 nm $E^1_1 = 200$

max 243 nm $E^1_1 = 287$ $\varepsilon = 12800$

infl 270 nm $E^1_1 = 37$

Stade B: $9\alpha$ fluoro $11\beta$, $17\beta$ dihydroxy $17\alpha$ [(3-trifluoro méthyl)phényl] androst 4-ene 3-one.

On refroidit à −35°C. 18 cm3 de complexe acide fluorhydrique-diméthylformamide, ajoute en 15 minutes 9 g de produit préparé au stade A, agite 10 minutes, ajoute à nouveau 4,5 cm3 de complexe, agite 30 minutes, verse le mélange réactionnel dans 500 cm3 d'un mélange d'eau, de glace et de 54 cm3 d'ammoniaque.

On agite 30 minutes, essore, lave à l'eau jusqu'à neutralité, sèche et obtient 9,65 g de produit brut que l'on purifie par chromatographie sur silice en éluant avec le mélange benzène-acétate d'éthyle 5—5; au produit obtenu on ajoute 50 cm3 d'éther isopropylique, glace, essore, lave à l'éther isopropylique, sèche, dissout le produit dans 30 cm3 de chlorure de méthylène, filtre à chaud, concentre, glace, essore, lave à l'éther isopropylique, sèche et obtient 4,34 g de produit attendu. P.F. = 254°C.

*Analyse:* $C_{26}H_{30}O_3F_4 = 466{,}50$ Calculé: C% 66,93 H% 6,48 F% 16,29

Trouvé: 67 6,6 16

*Spectre I.R.* (chloroforme) OH 3614 $cm^{-1}$ et 3594 $cm^{-1}$

C=O 1665 $cm^{-1}$

C=C 1624 $cm^{-1}$

*Spectre U.V.* (éthanol) infl: 224 nm $E^1_1 = 270$

max: 240 nm $E^1_1 = 350$ $\varepsilon = 16300$

Infl: 270 nm $E^1_1 = 17$

$[\alpha]_D = + 94°5 \pm 2°5$ (0,5% éthanol)

Exemple 12

*$9\alpha$ fluoro $11\beta$, $17\beta$-dihydroxy $17\alpha$ phénylméthyl androst 4-ène 3-one.*

Stade A: $9\beta$, $11\beta$ époxy $17\beta$ hydroxy $17\alpha$ phénylméthyl androst 4-en 3-one

Dans 300 cm3 d'éther éthylique anhydre, on introduit 13 g de lithium en poudre, refroidit à −10°C., ajoute en une heure 40 minutes, la solution de 22,75 g d'éther dibenzylique dans 275 cm3 d'éther éthylique anhydre, agite une heure et obtient le benzyllithium (titre 0,22 mole/litre).

Dans 485 cm3 de ce réactif, on introduit une solution de 7 g de 3-éthoxy $9\beta$, $11\beta$-époxy androsta 3,5-diène 17-one dans 50 cm3 d'éther éthylique anhydre, agite 2 heures 30 minutes à 0°C., ajoute 50 cm3 d'une solution aqueuse de chlorure d'ammonium, sépare la phase organique par décantation, lave à trois reprises avec 100 cm3 d'eau, filtre, sèche et concentre à sec. On obtient le 3-éthoxy $9\beta$, $11\beta$-époxy $17\beta$-hydroxy $17\alpha$-phényl méthyl androsta 3,5-diène que l'on reprend par 140 cm3 d'éthanol et 10 cm3 d'acide sulfurique N et chauffe à 60°C. pendant 30 minutes. On ajoute 100 cm3 de solution aqueuse à 5% de bicarbonate de sodium. Après 15 heures à une température comprise entre 0°C et +5°C., on essore, lave à l'eau, sèche et obtient 6,36 g de produit attendu. P.F. = 250°C.

*Spectre I.R.* (chloroforme) OH 3580 $cm^{-1}$ 3605 $cm^{-1}$

aromatique 494 $cm^{-1}$

Stade B: 9α-fluoro 11β, 17β-dihydroxy 17α-phénylméthyl androst 4-ène 3-one.

Dans 30 cm3 de complexe acide fluorhydrique-diméthylformamide, refroidi à −35°C., on introduit en 15 minutes, 5 g de 9β, 11β-époxy 17β-hydroxy 17α-phénylméthyl androst 4-ène 3-one, obtenu au stade A, agite 4 heures la solution obtenue, dilue avec 10 cm3 de réactif fluorant, agite encore une heure, verse dans 600 cm3 d'eau glacée additionnée de 200 cm3 de potasse 12N, agite 15 minutes, essore, lave à l'eau jusqu'à neutralité, sèche et obtient 5,8 g de produit brut. On purifie par chromatographie sur silice en éluant avec le mélange benzène-acétate d'éthyle (6—4), dissout le produit obtenu dans le chlorure de méthylène, ajoute 25 cm3 de diméthoxypropane, filtre et chasse le chlorure de méthylène. On refroidit,essore, lave au diméthoxypropane glacé, sèche et obtient 2,49 g de produit. P.F. = 218°C.

*Analyse:* $C_{26}$ $H_{33}$ $O_3F$ = 412,54

| | | | |
|---|---|---|---|
| calculé | C% = 75,69 | H% = 8,06 | F% = 4,60 |
| trouvé | 75,8 | 7,9 | 4,3 |

$[\alpha]_D$ = + 50°5 ± 2°5 (C = 0,6% chloroforme)

| | | | | |
|---|---|---|---|---|
| *Spectre IR* (chloroforme) | C = O | 1663 $cm^{-1}$ | | |
| | C = C | 1620 $cm^{-1}$ | 1603 $cm^{-1}$ | 1493 $cm^{-1}$ |
| | OH | 3610 $cm^{-1}$ | 3580 $cm^{-1}$ | |

| | | | |
|---|---|---|---|
| *Spectre UV* (éthanol) | max = 218 nm | $E_1^1$ = 290 | |
| | max = 238 nm | $E_1^1$ = 400 | ε = 16800 |

Exemple 13

*Etude pharmacologique du 11β, 17β-dihydroxy 17α-o-hydroxy phényl androsta 1,4-dien 3-one (produit A).*

1) Etude de l'activité antiinflammatoire par voie orale.

Elle a été recherchée selon le test classique du granulome. Dans la technique utilisée, modification de la méthode de R. MEIER et COLL. (Experientia, 1950, 6, 469), des rats Wistar conventionnels femelles, pesant de 100 à 110 g reçoivent une implantation de deux pellets de coton de 10 mg chacun sous la peau du thorax; le traitement oral, qui commence aussitôt après cette implantation, dure deux jours, à raison de deux administrations par jour. Seize heures après la dernière ingestion, soit le troisième jour, les animaux sont sacrifiés.

Les pellets, entourés du tissu de granulome formé, sont pesés à l'état frais, puis après séjour de dix huit heures à 60°C: le poids du granulome est obtenu par déduction du poids initial du coton.

La pesée des thymus, prélevés en même temps que les granulomes, permet d'apprécier l'activité thymolytique des produits.

Les résultats exprimés en DA 50 (c'est-à-dire en dose provoquant une inhibition du granulome de 50% et en dose provoquant une involution du thymus de 50%) sont les suivants:

| | granulome | Thymus |
|---|---|---|
| produit A | 3 mg/kg | 7 mg/kg |

CONCLUSION: le produit A présente sur le test classique du granulome une nette activité antiinflammatoire.

Exemple 14

*exemples de compositions pharmaceutiques*

On a préparé des comprimés répondant à la formule suivante:

— comprimés à 5 mg de principe actif
 produit A = 5 mg
 excipient (talc, amidon, stéarate de magnésium) qs pour 1 comprimé

— comprimés à 25 mg de principe actif
 produit A = 25 mg
 excipient (talc, amidon, stéarate de magnésium) qs pour 1 comprimé.

**Revendications**

1. Les composés de formule I:

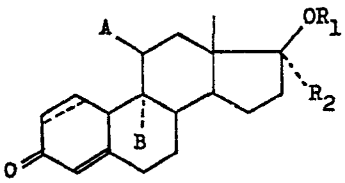

I

dans laquelle $R_1$ représente un atome d'hydrogène ou un radical acyle renfermant de 1 à 18 atomes de carbone choisi dans le groupe formé par les restes des acides alcanoïques, hydroxyalcanoïques, cycloalcoylcarboxyliques, cycloalcoylalcanoïques, de l'acide benzoïque, des acides phénylalcanoïques, des aminoacids, ou de l'acide formique, $R_2$ représente un radical aryle ou aralcoyle renfermant de 6 à 12 atomes de carbone ou un radical hétéroaryle, choisi dans le groupe constitué par les radicaux furyle, thiényle, pyridyle et pyrimidyle, les dits radicaux aryle, aralcoyle ou hétéroaryle étant éventuellement substitués par un ou plusieurs radicaux hydroxy, alcoyles ou alcoxy ayant de 1 à 4 atomes de carbone, par un ou plusieurs atomes d'halogène par un radical trifluorométhyle ou par une combinaison de ces divers substituants, les traits pointillés dans le cycle A représentent une double liaison éventuelle en 1(2) et, ou bien A représente un groupement oxo et B représente un atome d'hydrogène, ou bien A représente un groupement hydroxyle en position B et B représente un atome d'halogène ou un atome d'hydrogène, ou bien A et B forment ensemble une double liaison 9(11).

2. Les composés de formule I, tels que définis à la revendication 1, pour lesquels $R_2$ représente un radical aryle et notamment le radical phényle ou un radical aralcoyle et notamment le radical benzyle, éventuellement substitués par un groupement hydroxyle ou un groupement $CF_3$.

3. Les composés de formule I, tels que définis à la revendication 1 ou 2, pur lesquels $R_1$ représente un atome d'hydrogène.

4. Les composés de formule I, tels que définis à l'une quelconque des revendications 1 à 3, pour lesquels A représente un groupement oxo et B un atome d'hydrogène.

5. Les composés de formule I, tels que définis à l'une quelconque des revendications 1 à 3, pour lesquels A représente un groupement OH et B un atome d'hydrogène.

6. Les composés de formule I tels que définis à l'une quelconque des revendications 1 à 3, pour lesquels A représente un groupement OH et B un atome d'halogène comme par exemple un atome de fluor.

7. Les composés de formule I tels que définis à l'une quelconque des revendications 1 à 3, pour lesquels A et B forment ensemble une double liaison éthylénique en 9 (11).

8. Les composés de formule I tels que définis à l'une quelconque des revendications 1 à 7, pour lesquels le cycle A ne porte pas d'insaturation éthylénique en 1 (2).

9. Les composés de formule I tels que définis à l'une quelconque des revendications 1 à 7, pour lesquels le cycle A porte une insaturation en 1 (2).

10. La 11$\beta$, 17$\beta$-dihydroxy 17$\alpha$ o-hydroxyphényl androsta 1,4-dien 3-one.

11. Procédé de préparation des composés de formule I, tels que définis à la revendication 1, caractérisé en ce que l'on soumet un composé de formule

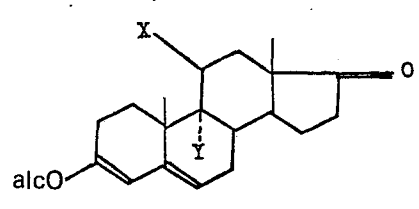

II

dans laquelle alc représente un radical alcoyle renfermant de 1 à 6 atomes de carbone, et ou bien X représente un groupement oxo et Y représente un atome d'hydrogène, ou bien X et Y forment ensemble une double liaison éthylénique en 9(11), à l'action d'un composé de formule $R_2$ Li, dans laquelle $R_2$ conserve la même signification que dans la revendication 1, puis à celle d'un agent d'hydrolyse acide, pour obtenir le composé de formule

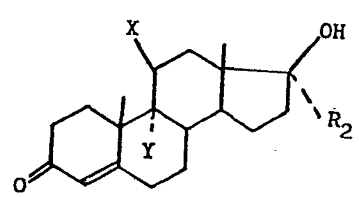

IA

que l'on soumet, si désiré, dans le cas où X représente un groupement oxo et Y représente un atome d'hydrogène, à l'action d'un orthoformiate d'alcoyle en milieu acide, puis à l'action d'un agent de réduction et enfin à celle d'un agent d'hydrolyse acide, pour obtenir un composé de formule

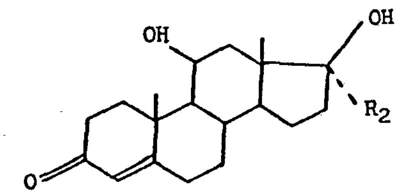

IB

ou que l'on soumet, dans le cas où X et Y forment ensemble und double liaison éthylénique en 9(11), à l'action du N-bromosuccinimide en présence d'acide perchlorique, pour obtenir un composé de formule

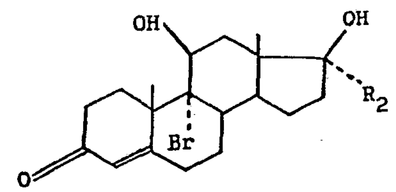

IC

que l'on soumet si désiré a l'action d'un agent basique, pour obtenir un composé de formule

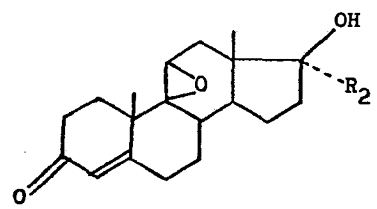

III

que l'on soumet à l'action d'un hydracide de formule H Hal dans laquelle Hal représente un atome de chlore ou de fluor, pour obtenir un composé de formule

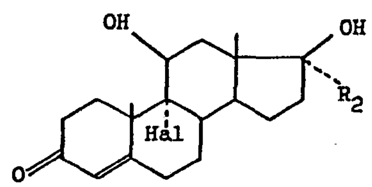

ID

puis si désiré, soumet chacun des composés obtenus, IA, IB, IC ou ID à l'action d'un agent de deshydrogénation, pour obtenir les composés Δ1,4 correspondants, et soumet enfin, si désiré, chacun des composés obtenus, répondant à la formule I dans laquelle $R_1$ représente un atome d'hydrogène, à l'action d'un agent d'estérification sélectif, pour obtenir les composés correspondants répondant à la formule I dans laquelle $R_1$ représente un radical acyle.

12. Procédé selon la revendication 11, caractérisé en ce que le composé de formule III est obtenu en soumettant un composé de formule

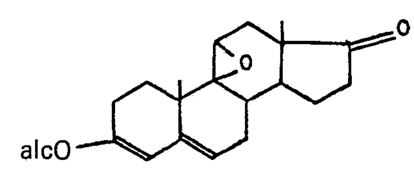

IV

dans laquelle alc représente un radical alcoyle renfermant 1 à 6 atomes de carbone, à l'action d'un composé de formule $R_2$ Li dans laquelle $R_2$ conserve la même signification que dans la revendication 1, puis à celle d'un agent d'hydrolyse acide, pour obtenir le composé de formule III désiré.

13. A titre de médicament, les composés définis à l'une quelconque des revendications 1 à 9.

14. A titre de médicament, le composé de la revendication 10.

15. Les compositions pharmaceutiques renfermant comme principe actif au moins un médicament défini à la revendication 13 ou 14.

**Claims**

1. The compounds with the formula I

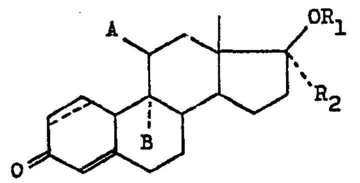

I

in which $R_1$ represents a hydrogen atom or an acyl radical containing from 1 to 18 carbon atoms chosen from the group formed by the residues of alkanoic, hydroxyalkanoic, cycloalkylcarboxylic, and cycloalkylalkanoic acids, of benzoic acid, of the phenylalkanoic acids, of the amino acids, or of formic acid, $R_2$ represents an aryl or aralkyl radical containing from 6 to 12 carbon atoms or a heteroaryl radical chosen from the group constituted by the furyl, thienyl, pyridyl and pyrimidyl radicals, the said aryl, aralkyl or heteroaryl radicals possibly being substituted by one or more hydroxy, alkyl or alkoxy radicals having from 1 to 4 carbon atoms, by one or more halogen atoms, by a trifluoromethyl radical or by a combination of these different substituents, the broken lines in the ring A representing a possible double bond at 1(2) and, either A represents an oxo group and B represents a hydrogen atom or A represents a hydroxyl group in position β and B represents a halogen atom or a hydrogen atom, or A and B together form a double bond 9(11).

2. The compounds with the formula I, as defined in Claim 1, for which $R_2$ represents an aryl radical and in particular a phenyl radical, or an aralkyl radical and in particular a benzyl radical, possibly substituted by a hydroxyl group or a $CF_3$ group.

3. The compounds with the formula I, as defined in Claim 1 or 2, for which $R_1$ represents a hydrogen atom.

4. The compound with the formula I, as defined in any one of the Claims 1 to 3, for which A represents an oxo group and B a hydrogen atom.

5. The compounds with the formula I, as defined in any one of the Claims 1 to 3, for which A represents an OH group and B a hydrogen atom.

6. The compounds with the formula I, as defined in any one of the Claims 1 to 3, for which A represents an OH group and B a halogen atom such, for example, as a fluorine atom.

7. The compounds with the formula I as defined in any one of the Claims 1 to 3, for which A and B together form an ethylene double bond at 9(11).

8. The compounds with the formula I as defined in any one of the Claims 1 to 7, for which ring A does not carry an ethylene unsaturation at 1(2).

9. The compounds with the formula I as defined in any one of the Claims 1 to 7, for which ring A carries an unsaturation at 1(2).

10. 11β,17β-dihydroxy 17α o-hydroxyphenyl androsta 1,4-dien 3-one.

11. Process for the preparation of the compounds with the formula I, as described in Claim 1, characterized in that a compound with the formula

II

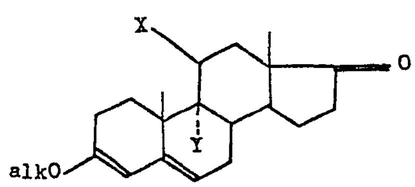

In which alk represents an alkyl radical containing from 1 to 6 carbon atoms and, either X represents an oxo group and Y represents a hydrogen atom, or X and Y together form an ethylene double bond at 9(11), is submitted to the action of a compound with the formula $R_2$ Li, in which $R_2$ retains the same significance as in Claim 1, then to that of an acid hydrolysis agent, so as to obtain the compound with the formula

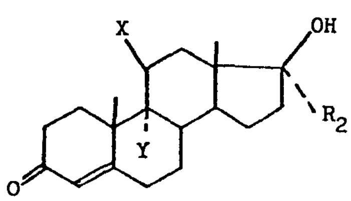

IA

which, if desired, in the case where X represents an oxo group and Y represents a hydrogen atom, is submitted to the action of an alkyl orthoformate in an acid medium, then to the action of a reducing agent, and finally to that of an acid hydrolysis agent, so as to obtain a compound with the formula

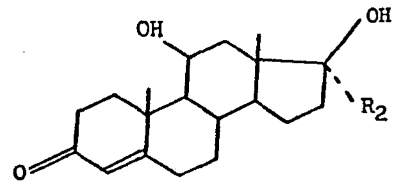

IB

or in the case where X and Y together form an ethylene double bond at 9(11), is submitted to the action of N-bromosuccinimide in the presence of perchloric acid, so as to obtain a compound with the formula

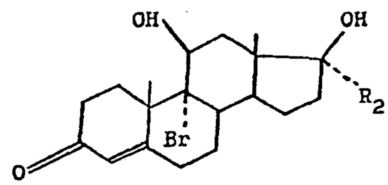

IC

which if desired is submitted to the action of a basic agent, so as to obtain a compound with the formula

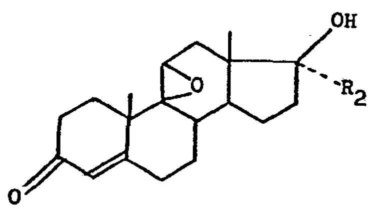

III

which is submitted to the action of a hydracid with the formula H Hal in which Hal represents a chlorine or a fluorine atom, so as to obtain a compound with the formula

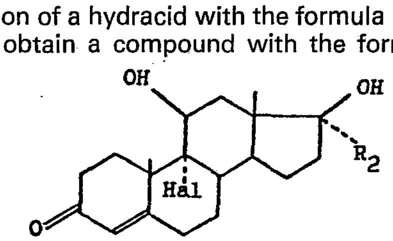

ID

then, if desired, each of the compounds obtained IA, IB, IC or ID is submitted to the action of a dehydrogenation agent, so as to obtain the corresponding Δ1,4 compounds, and finally, if desired, each of the compounds obtained, corresponding to the formula I in which $R_1$ represents a hydrogen atom, is submitted to the action of a selective esterification agent, so as to obtain the corresponding compounds responding to the formula I in which $R_1$ represents an acyl radical.

12. Process according to Claim 11, characterized in that the compound with the formula III is obtained by submitting a compound with the formula

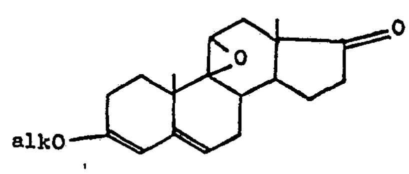

IV

in which alk represents an alkyl radical containing 1 to 6 carbon atoms, is submitted to the action of a compound with the formula $R_2$ Li in which $R_2$ retains the same significance as in Claim 1, then to that of an acid hydrolysis agent, so as to obtain the desired compound with the formula III.

13. As medicaments, the compounds defined in any one of the Claims 1 to 9.

14. As medicaments, the compound of Claim 10.

15. The pharmaceutical compositions containing as active principle at least one medicament defined in Claim 13 or 14.

**Patentansprüche**

1. Verbindungen der Formel I

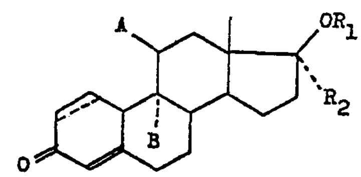

I

worin $R_1$ ein Wasserstoffatom oder einen Acylrest mit 1 bis 18 Kohlenstoffatomen, ausgewählt unter den Resten der Alkansäuren, Hydroxyalkansäuren, Cycloalkylcarbonsäuren, Cycloalkylalkansäuren, der Benzoesäure, der Phenylalkansäuren, der Aminosäuren oder der Ameisensäure bedeutet, $R_2$ einen Aryl- oder Aralkylrest mit 6 bis 12 Kohlenstoffatomen oder einen Heteroarylrest, ausgewählt unter den Furyl-, Thienyl-, Pyridyl- und Pyrimidylresten bedeutet, wobei die Aryl-, Aralkyl- oder Heteroarylreste gegebenenfalls durch ein oder mehrere Hydroxy-, Alkyl- oder Alkoxyreste mit 1 bis 4 Kohlenstoffatomen, durch ein oder mehrere Halogenatome, durch einen Trifluormethylrest oder durch eine Kombination dieser verschiedenen Substituenten substituiert sind, die gestrichelten Linien in dem Ring A eine etwaige Doppelbindung in 1,2-Stellung bedeuten und entweder A eine Oxo-Gruppe bedeutet und B ein Wasserstoffatomen bedeutet oder A eine Hydroxylgruppe in $\beta$-Stellung bedeutet und B ein Halogenatom oder ein Wasserstoffatom bedeutet oder A und B gemeinsam eine 9(11)-Doppelbindung bilden.

2. Verbindungen der Formel I gemäß Anspruch 1, worin $R_2$ einen Arylrest und insbesondere den Phenylrest oder einen Aralkylrest und insbesondere den Benzylrest, die gegebenenfalls durch eine Hydroxylgruppe oder $CF_3$-Gruppe substituiert sind, bedeutet.

3. Verbindungen der Formel I gemäß Anspruch 1 oder 2, worin $R_1$ ein Wasserstoffatom bedeutet.

4. Verbindungen der Formel I gemäß einem der Ansprüche 1 bis 3, worin A eine Oxo-Gruppe bedeutet und B ein Wasserstoffatom darstellt.

5. Verbindungen der Formel I gemäß einem der Ansprüche 1 bis 3, worin A eine OH-Gruppe bedeutet und B ein Wasserstoffatom darstellt.

6. Verbindungen der Formel I gemäß einem der Ansprüche 1 bis 3, worin A eine OH-Gruppe bedeutet und B ein Halogenatom, z.B. ein Fluoratom, darstellt.

7. Verbindungen der Formel I gemäß einem der Ansprüche 1 bis 3, worin A und B gemeinsam eine äthylenische Doppelbindung in 9(11)-Stellung bilden.

8. Verbindungen der Formel I gemäß einem der Ansprüche 1 bis 7, worin der Ring A keine äthylenische Unsättigung in 1(2)-Stellung enthält.

9. Verbindungen der Formel I gemäß einem der Ansprüche 1 bis 7, worin der Ring A eine Unsättigung in 1(2)-Stellung enthält.

10. 11$\beta$,17$\beta$-Dihydroxy-17$\alpha$-o-hydroxyphenylandrosta-1,4-dien-3-on.

11. Verfahren zur Herstellung der Verbindungen der Formel I gemäß Anspruch 1, dadurch gekennzeichnet, daß man eine Verbindung der Formel

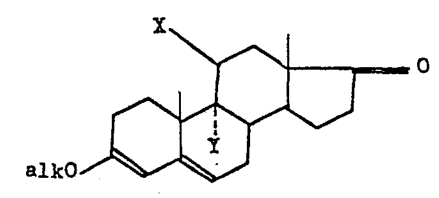

II

worin Alk einen Alkylrest mit 1 bis 6 Kohlenstoffatomen bedeutet und entweder X eine Oxo-Gruppe darstellt und Y ein Wasserstoffatom bedeutet oder X und Y gemeinsam eine äthylenische Doppelbindung in 9(11)-Stellung bilden, der Einwirkung einer Verbindung der Formel $R_2Li$, worin $R_2$ die in in Anspruch 1 angegebene Bedeutung besitzt, und danach derjenigen eines sauren Hydrolysemittels unterzieht, um die Verbindung der Formel

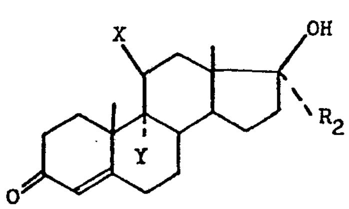

IA

zu erhalten, die man gewünschtenfalls, wenn X eine Oxo-Gruppe bedeutet und Y ein Wasserstoffatom darstellt, der Einwirkung eines Alkylorthoformiats in saurem Milieu und danach der Einwirkung eines

Reduktionsmittels und schließlich derjenigen eines sauren Hydrolysemittels unterzieht, um eine Verbindung der Formel

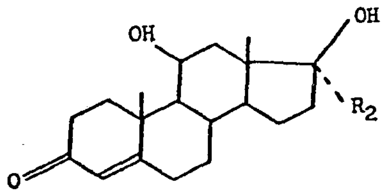

IB

zu erhalten oder die man, wenn X und Y gemeinsam eine äthylenische Doppelbindung in 9(11)-Stellung bilden, der Einwirkung von N-Bromsuccinimid in Gegenwart von Perchlorsäure unterzieht, um eine Verbindung der Formel

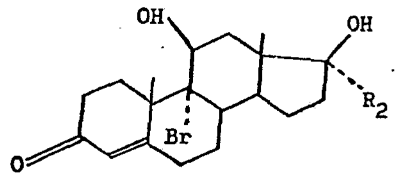

IC

zu erhalten, die man gewünschtenfalls der Einwirkung eines basischen Mittels unterzieht, um eine Verbindung der Formel

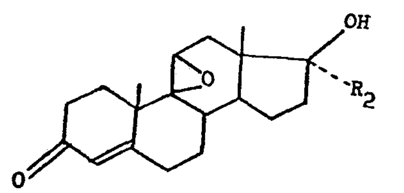

III

zu erhalten, die man der Einwirkung eines sauren Hydrolysemittels der Formel HHal, worin Hal ein Chlor- oder Fluor-atom bedeutet, unterzieht, um eine Verbindung der Formel

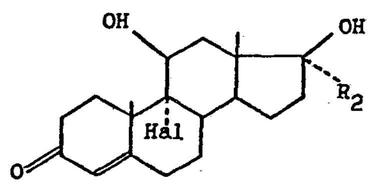

ID

zu erhalten, wonach man gewünschtenfalls jede der erhaltenen Verbindungen IA, IB, IC oder ID der Einwirkung eines Dehydrierungsmittels unterzeiht, um die entsprechenden Δ 1,4-Verbindungen zu erhalten und schließlich gewünschtenfalls jede der erhaltenen Verbindungen entsprechend der Formel I, worin $R_1$ ein Wasserstoffatom bedeutet, der Einwirkung eines selektiven Veresterungsmittels unterzieht, um die entsprechenden Verbindungen der Formel I zu erhalten, worin $R_1$ Acylrest bedeutet.

12. Verfahren gemäß Anspruch 11, dadurch gekennzeichnet, daß die Verbindung der Formel III erhalten wird, indem man eine Verbindung der Formel

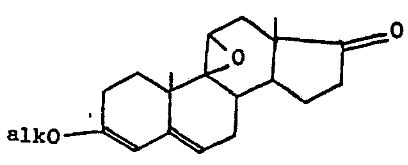

IV

worin Alk einen Alkylrest mit 1 bis 6 Kohlenstoffatomen bedeutet, der Einwirkung einer Verbindung der Formel $R_1Li$, worin $R_2$ die in Anspruch 1 angegebene Bedeutung besitzt, und danach derjenigen eines sauren Hydrolysemittels unterzieht, um die gewünschte Verbindung der Formel III zu erhalten.

13. Als Arzneimittel die Verbindung gemäß einem der Ansprüche 1 bis 9.

14. Als Arzneimittel die Verbindung gemäß Anspruch 10.

15. Pharmazeutische Zusammensetzungen, enthaltend als Wirkstoff zumindest ein Arzneimittel gemäß Anspruch 13 oder 14.